# EUROPÄISCHE PATENTSCHRIFT

(11) **EP 0 799 470 B1**
(45) Veröffentlichungstag und Bekanntmachung des Hinweises auf die Patenterteilung: **22.07.1998**
(21) Anmeldenummer: 95940963.2
(22) Anmeldetag: 15.12.1995
(51) Int. Cl.: G10K 11/34

(54) **VORRICHTUNG ZUR BEHANDLUNG EINES OBJEKTES MIT FOKUSSIERTEN ULTRASCHALLWELLEN**
SYSTEM FOR PROCESSING AN OBJECT WITH FOCUSED ULTRASONIC WAVES
SYSTEME POUR TRAITER UN OBJET A L'AIDE D'ONDES ULTRASONORES FOCALISEES

(30) Priorität: 23.12.1994 DE 4446429
(43) Veröffentlichungstag der Anmeldung: 08.10.1997
(73) Patentinhaber: SIEMENS AKTIENGESELLSCHAFT, 80333 München (DE)
(72) Erfinder: SCHÄTZLE, Ulrich, D-91341 Röttenbach (DE); SCHMIDT, Erhard, D-91054 Erlangen (DE)
(86) Internationale Anmeldenummer: DE9501805
(87) Internationale Veröffentlichungsnummer: WO9620471

(56) Entgegenhaltungen:
- EP-A- 0 104 928
- DE-A- 3 048 527
- DE-A- 3 236 218
- DE-A- 3 330 698
- DE-C- 4 302 538
- US-A- 4 159 462
- US-A- 5 065 741
- US-A- 5 158 071
- PATENT ABSTRACTS OF JAPAN vol. 018, no. 334 (C-1216), 24.Juni 1994 & JP,A,06 078922 (FUJITSU LTD), 22.März 1994,

## Beschreibung

Die Erfindung betrifft eine Vorrichtung zur Behandlung eines Objektes mit fokussierten Ultraschallwellen, aufweisend einen aus einer Vielzahl von Ultraschallwandlerelementen zusammengesetzten Ultraschallwandlern und eine Einrichtung zur Ansteuerung des Ultraschallwandlers, die den Ultraschallwandlerelementen des Ultraschallwandlers einer gewünschten Position des Fokus entsprechend zeitlich zueinander versetzte elektrische Signale zuführt.

Derartige Vorrichtungen werden beispielsweise zur Behandlung von pathologischen Gewebeveränderungen verwendet. Dabei wird das pathologische Gewebe durch als therapeutische akustische Wellen abgestrahlte fokussierte Ultraschallwellen erwärmt. Sofern die auftretenden Temperaturen unterhalb von 45 °C liegen, wird der Zellstoffwechsel mit der Folge gestört, daß im Falle von Tumoren eine Verlangsamung des Wachstums oder sogar ein Rückgang des Tumors eintritt. Diese Behandlungsart ist als lokale Hyperthermie bekannt. Werden Temperaturen jenseits von 45 °C erreicht, koaguliert das Zelleiweiß mit der Folge der Nekrotisierung des Gewebes. Letztere Behandlungsart wird als Thermotherapie bezeichnet.

Die therapeutischen akustischen Wellen werden als Dauerschall oder gepulster Dauerschall abgestrahlt.

Therapiegeräte der eingangs genannten Art können auch bei der Behandlung von Steinleiden (Lithotripsie) und Knochenleiden (Osteorestauration) Verwendung finden. Die therapeutischen akustischen Wellen werden dann in Form von Stoßwellen abgestrahlt.

Die Möglichkeit, den Fokus der akustischen Wellen verlagern zu können, ist vorgesehen, um eine Verlagerung des Fokus innerhalb des zu behandelnden Objektes zu ermöglichen, ohne daß der Ultraschallwandler und das zu behandelnde Objekt relativ zueinander verschoben werden müssen.

Eine Vorrichtung der eingangs genannten Art ist beispielsweise aus der DE 43 02 538 C1 bekannt. Bei dieser Vorrichtung wird die Verlagerbarkeit des Fokus dadurch realisiert, daß jedem der Ultraschallwandlerelemente ein Verzögerungsglied mit einstellbarer Verzögerungszeit zugeordnet ist. Die Verzögerungsglieder dienen dazu, die den Ultraschallwandlerelementen von einem einzigen Oszillator zur Ultraschall-Erzeugung zugeführten Signale derart relativ zueinander zu verzögern bzw. in ihrer Phasenlage zu verändern, daß sich die jeweils gewünschte Fokuslage ergibt. Die Verzögerungszeiten der Verzögerungsglieder werden mittels einer Steuerungseinheit eingestellt. Es muß also ein ganz erheblicher technischer Aufwand getrieben werden, der mit entsprechend hohen Kosten verbunden ist. Außerdem ist mit dem aufwendigen Aufbau der Elektronik ein hoher Raumbedarf verbunden.

Während im Falle der DE 43 02 538 C1 für alle Ultraschallwandlerelemente ein gemeinsamer Oszillator die zur Ultraschall-Erzeugung erforderlichen Signale liefert, ist im Falle einer aus der US 5 065 741 bekannten Vorrichtung jedem Ultraschallwandlerelement ein Ansteuerschaltkreis zugeordnet, der dem jeweiligen Ultraschallwandlerelement die zur Ultraschall-Erzeugung erforderlichen Signale liefert, wobei die Zeitpunkte der Aktivierung der Ansteuerschaltkreise mittels in Abhängigkeit von der Auswertung mittels einer Ultraschall-Ortungseinrichtung erzeugter Ultraschall-Bilder von einem Rechner eingestellt werden, dem ein Speicher zugeordnet ist.

Auch die US 5 158 071 betrifft eine Vorrichtung, bei der jedem Ultraschallwandlerelement ein Ansteuerschaltkreis zugeordnet, der die zur Ultraschall-Erzeugung erforderlichen Signale für das jeweilige Ultraschallwandlerelement liefert, wobei die Zeitpunkte der Aktivierung der Ansteuerschaltkreise in Abhängigkeit von der jeweils gewünschten Position des Fokus eingestellt wird.

Die DE 30 48 527 A1 betrifft einen diagnostischen Ultraschall-Applikator bei dem ein gemeinsamer Oszillator die zur Ultraschall-Erzeugung erforderlichen Signale für mehrere Gruppen von Ultraschallwandlerelementen liefert. Dabei werden die Signale des Oszillators den einzelnen Gruppen über entsprechende Ansteuerschalter zugeführt, die mittels eines Digitalsteuergliedes, z.B. eines 8 bit D-Registers, aktiviert werden.

Die DE 32 36 218 A1 betrifft einen diagnostischen Ultraschall-Applikator bei dem eine Demultiplexer/Sendeeinheit die zur Ultraschall-Erzeugung erforderlichen Signale für die Ultraschallwandlerelementen liefert. Dabei werden diese Signale den Ultraschallwandlerelementen in Abhängigkeit von in einem Speicher abgespeicherten Verzögerungszeiten zugeführt.

Der Erfindung liegt die Aufgabe zugrunde, eine Vorrichtung der eingangs genannten Art so auszubilden, daß die Verlagerung des Fokus der akustischen Wellen auf technisch einfache und kostengünstige Weise ermöglicht ist.

Nach der Erfindung wird diese Aufgabe gelöst durch eine Vorrichtung zur Behandlung eines Objektes mit fokussierten Ultraschallwellen, aufweisend
- einen aus einer Vielzahl von Ultraschallwandlerelementen zusammengesetzten Ultraschallwandler, und
- eine Einrichtung zur Ansteuerung des Ultraschallwandlers, welche für jedes Ultraschallwandlerelement einen Speicher aufweist, in dem im Betrieb ein einer gewünschten Position des Fokus der Ultraschallwellen entsprechendes bit-Muster gespeichert ist, und eine Steuereinheit, die die einzelnen Speicher parallel derart zyklisch adressiert, daß an den Datenausgängen der Speicher entsprechend der gewünschten Position des Fokus zeitlich zueinander versetzte elektrische Signale auftreten, die dem jeweiligen Ultraschallwandlerelement zugeführt sind.

Im Falle der erfindungsgemäßen Vorrichtung werden den Ultraschallwandlerelementen als zur Ultraschall-Erzeugung dienende Signale also nicht die Ausgangssignale eines Oszillators oder dergleichen zugeführt. Vielmehr werden den Ultraschallwandlerelementen die an den Datenausgängen der Speicher zur Verfügung stehenden Signale als als zur Ultraschall-Erzeugung dienende Signale zugeführt. Zur Realisierung der Verlagerung des Fokus wird im Falle der erfindungsgemäßen Vorrichtung also lediglich eine der Anzahl der Ultraschallwandlerelemente entsprechende Anzahl von Speichern und eine die Speicher adressierende Steuereinheit benötigt. Bei den Speichern und den wesentlichen Bauelementen der Steuereinheit handelt es sich um kostengünstig in Form von integrierten Schaltungen erhältliche Halbleiterbauelemente, so daß sich gegenüber herkömmlichen Vorrichtungen ein einfacherer, kostengünstigerer und auch Bauraum sparender Aufbau ergibt. In diesem Zusammenhang ist es wesentlich, daß im Gegensatz zum Stand der Technik eine Einstellung von Verzögerungszeiten nicht stattfinden muß, da die Phasenlage der Signale relativ zueinander durch die bit-Muster vorgegeben ist.

Gemäß einer besonders bevorzugten Ausführungsform der Erfindung enthält die Vorrichtung als Speicher Schreib-/Lesespeicher, in die die Steuereinheit die der gewünschten Position des Fokus entsprechenden bit-Muster einschreibt. Auf diese Weise ist es möglich, unterschiedliche Positionen des Fokus einzustellen, ohne daß die Speicher ausgetauscht werden müssen. In diesem Zusammenhang ist gemäß einer Variante der Erfindung vorgesehen, daß die Steuereinheit die einer gewünschten Position des Fokus entsprechenden bit-Muster errechnet und in den Speicher einschreibt. Gemäß einer besonders bevorzugten Ausführungsform der Erfindung ist jedoch vorgesehen, daß die Steuereinheit einen Datenspeicher aufweist, in dem für unterschiedliche Positionen des Fokus bit-Muster gespeichert sind, und daß die Steuereinheit die der jeweils gewünschten Position des Fokus entsprechenden bit-Musters in die Speicher einschreibt. Auf diese Weise ergibt sich ein gegenüber der zuerst genannten Möglichkeit vereinfachter Aufbau der Steuereinheit, da die Errechnung der bit-Muster entfallen kann. Die Anzahl der Positionen des Fokus bezüglich derer bit-Muster gespeichert sind ist praktisch nur durch die Kapazität des Datenspeichers begrenzt. Vorzugsweise sind die unterschiedlichen Positionen des Fokus für die in dem Datenspeicher bit-Muster gespeichert sind zwei- oder dreidimensional matrixartig angeordnet.

Der Steuereinheit sind gemäß einer Ausführungsform der Erfindung Eingabemittel für eine gewünschte Position des Fokus zugeordnet. Vorzugsweise weist die Vorrichtung eine Ortungseinrichtung auf, mittels derer ein Bild zumindest eines Bereichs des zu behandelnden Objektes erzeugbar ist, wobei die Eingabemittel derart ausgebildet sind, daß die jeweils gewünschte Position des Fokus in dem erzeugten Bild anwählbar ist. Für den Fall, daß die Steuereinheit nicht derart ausgebildet ist, daß sie die gewünschte Position des entsprechenden bit-Muster errechnet, ist dann vorgesehen, daß die Steuereinheit diejenigen in dem Datenspeicher gespeicherten bit-Muster in die Speicher einschreibt, die derjenigen Position des Fokus entsprechen, die am nächsten bei der mittels der Eingabemittel angegebenen gewünschten Position des Fokus liegt.

Obwohl grundsätzlich auch auf Röntgenbasis arbeitende Ortungseinrichtungen in Frage kommen, ist gemäß einer Ausführungsform der Erfindung eine Ultraschall-Ortungseinrichtung vorgesehen. Wenn die Ultraschall-Ortungseinrichtung ein Schnittbild zumindest eines Bereichs des zu behandelnden Objektes erzeugt, ist gemäß einer besonders bevorzugten Ausführungsform der Erfindung vorgesehen, daß die in dem Datenspeicher gespeicherten unterschiedlichen Positionen des Fokus in einer Ebene liegen, die in dem in dem Schnittbild abgebildeten Bereich des zu behandelnden Objektes enthalten ist. Auf diese Weise ist sichergestellt, daß die einstellbaren Positionen des Fokus tatsächlich in dem mittels der Ultraschall-Ortungseinrichtung erzeugten Ultraschallbild dargestellt sind, so daß die Gefahr, daß versehentlich der Fokus in eine Position gebracht wird, in der eigentlich keine Behandlung erfolgen soll, gering ist.

Besonders vorteilhaft ist es, wenn der an sich zur Erzeugung der der Behandlung dienenden fokussierten Ultraschallwellen vorgesehene Ultraschallwandler zugleich auch Bestandteil der Ultraschall-Ortungseinrichtung ist, da es sich dann erübrigt, zu Ortungszwecken einen besonderen Ultraschallwandler vorzusehen. Besonders günstig ist es in diesem Zusammenhang, wenn der Ultraschallwandler eine lineare Anordnung (linear array) von Ultraschallwandlerelementen enthält, da es dann möglich ist, das zu behandelnde Objekt zur Erzeugung eines Ultraschall-Schnittbildes in an sich bekannter Weise auf rein elektronischem Wege in einem linearen Abtastvorgang (linear scan) abzutasten.

Zwischen die Datenausgänge der Speicher und die entsprechenden Ultraschallwandlerelemente sind gemäß einer Variante der Erfindung Signalumformungsmittel geschaltet, die die an den Datenausgängen der Speicher auftretenden rechteckartigen Signale in wenigstens im wesentlichen sinusförmige Signale umwandeln. Obwohl also die den Ultraschallwandlern zugeführten elektrischen Signale auf digitalem Wege erzeugt werden, besteht somit dennoch die Möglichkeit, mit wenigstens im wesentlichen sinusförmigen Ultraschallwellen zu therapieren. Geringfügige Abweichungen der den Ultraschallwandlerelementen zugeführten elektrischen Signale von der Sinusform sind übrigens ohne Bedeutung, da infolge nicht linearer Effekte ohnehin Verzerrungen der Ultraschallwellen auf ihrem Weg zu den jeweils zu behandelnden Bereich auftreten.

Die Signalumformungsmittel lassen sich auf besonders einfache und kostengünstige Weise realisieren, wenn sie LC-Netzwerke enthalten.

Aus den vorstehenden Ausführungen ist klar, daß für die den einzelnen Ultraschallwandlerelementen zugeordneten Speicher eine Speichertiefe von 1 bit ausreicht. Die erforderliche Speicherlänge hängt davon ab, in welchem Maße eine Verlagerung der Position des Fokus gewünscht wird. Gemäß einer Variante der Erfindung weisen die Speicher jeweils eine Speicherlänge von 16 bit auf. Auf diese Weise läßt sich die Position des Fokus in einem für Behandlungen im Urogenitalbereich, insbesondere der Prostata, ausreichenden Maße verlagern.

Um eine optimale Funktion der Vorrichtung zu gewährleisten, muß die Taktfrequenz, mit der die Steuereinheit die Speicher adressiert gleich der mit der Speicherlänge multiplizierten Frequenz der Ultraschallwellen sein.

Um auch Ultraschallwellen größerer Intensität erzeugen zu können, sind gemäß einer Ausführungsform der Erfindung zwischen die Datenausgänge der Speicher und die entsprechenden Ultraschallwandlerelemente geschaltete Treiberstufen vorgesehen. Gemäß einer besonders bevorzugten Ausführungsform der Erfindung sind die Treiberstufen mit einer Stromversorgung verbunden, deren Ausgangsspannung mittels der Steuereinheit einstellbar ist. Es besteht also die Möglichkeit, die Amplitude der den Ultraschallwandlerelementen zugeführten elektrischen Signale und damit die Amplitude der erzeugten Ultraschallwellen den jeweiligen Bedürfnissen einzustellen.

Um im Interesse der Sicherheit des Patienten sicherstellen zu können, daß die Amplitude der Ultraschallwellen das jeweils gewünschte Maß nicht überschreitet, sind gemäß einer Variante der Erfindung Überwachungsmittel vorgesehen, die Spannung und/oder Strom an den Ausgängen der Treiberstufen und/oder an den Ausgängen der Signalumformungsmittel und/oder am Ausgang der Stromversorgung messen und mit einem entsprechenden Referenzwert bzw. entsprechenden Referenzwerten vergleichen.

Ebenfalls im Interesse der Sicherheit des Patienten ist gemäß einer Ausführungsform der Erfindung vorgesehen, daß die Steuereinheit in die zu im Bereich des Randes des Ultraschallwandlers liegenden Ultraschallwandlerelementen gehörigen Speicher von den übrigen Speichern derart abweichende bit-Muster einschreibt, daß die von dem im Bereich des Randes des Ultraschallwandlers liegenden Ultraschallwandlerelementen ausgehenden Ultraschallwellen eine im Vergleich zu dem von den übrigen Ultraschallwandlerelementen ausgehenden Ultraschallwellen verringerter Intensität aufweisen. Es werden auf diese Weise Interferenzerscheinungen vermieden, die zur Ausbildung von unerwünschten Nebenfoki führen können. Wenn die Steuereinheit in die zu nicht im Bereich des Randes des Ultraschallwandlers liegenden Ultraschallwandlerelementen gehörigen Speicher bit-Muster einschreibt, die jeweils eine Folge unmittelbar aufeinanderfolgender gesetzter bits enthalten, kann die verringerte Intensität der Ultraschallwellen im Bereich des Randes des Ultraschallwandlers auf einfache Weise realisiert werden, in dem in die zu im Bereich des Randes des Ultraschallwandlers liegenden Ultraschallwandlerelementen gehörigen Speicher bit-Muster eingeschrieben werden, die eine verringerte Anzahl von aufeinanderfolgenden gesetzten bits enthalten.

Die Erfindung wird nachfolgend am Beispiel einer in den Zeichnungen dargestellten erfindungsgemäßen Vorrichtung näher erläutert, die zur Behandlung der benignen Prostata-Hyperplasie vorgesehen ist. Es zeigen:
- Fig. 1: in perspektivischer Darstellung ein zu der Vorrichtung gehöriges Handstück, das den elektroakustischen Wandler enthält,
- Fig. 2: in perspektivischer Darstellung den in dem Handstück enthaltenen Ultraschallwandler,
- Fig. 3: die Vorrichtung in Form eines Blockschaltbildes, und
- Fig. 4: zur Ansteuerung unterschiedliche Ultraschallwandler der Vorrichtung bestimmte elektrische Signale.

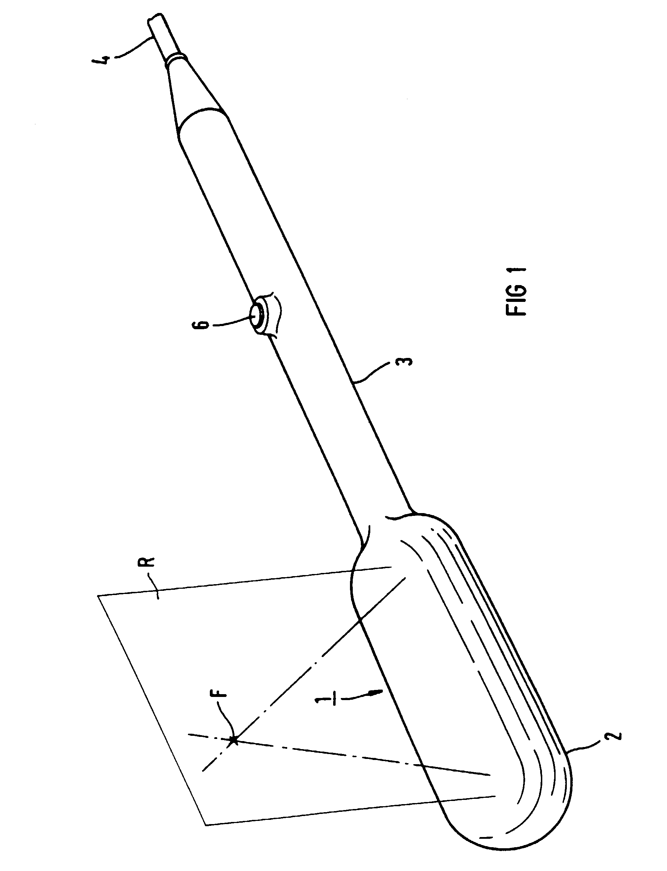

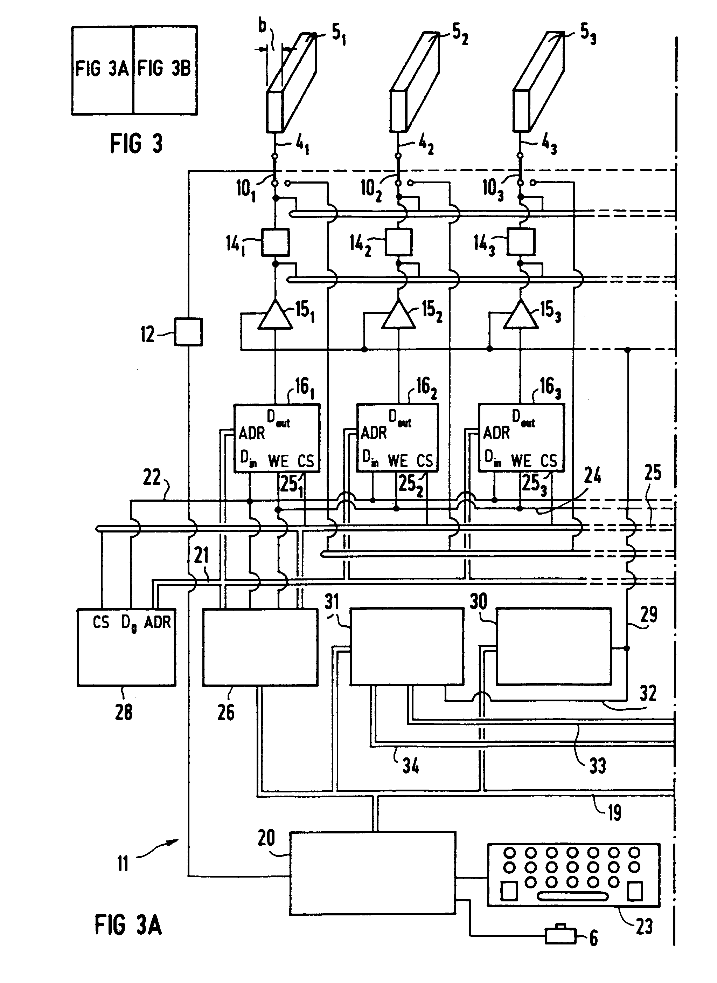

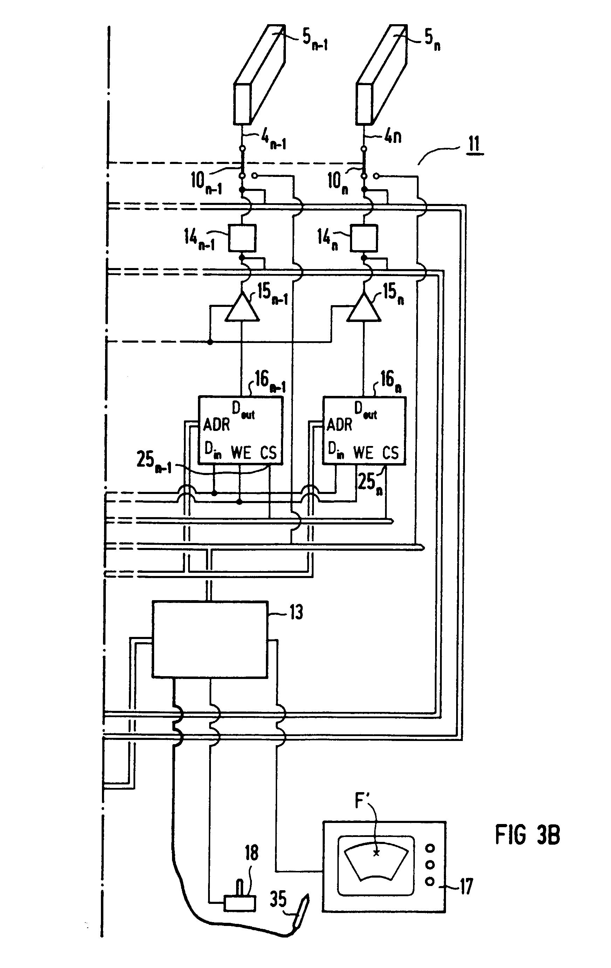

Die Fig. 1 zeigt von einer erfindungsgemäßen Vorrichtung zur Behandlung der benignen Prostata-Hyperplasie ein mit 1 bezeichnetes Handstück, das zur rektalen Applikation vorgesehen ist. Das Handstück 1 ist von etwa löffelförmiger Gestalt und weist ein etwa ovales, flaches Applikationsende 2 auf, an dem ein Handgriff 3 angebracht ist. Das Applikationsende, das eine Dicke von etwa 15 mm, eine Breite von etwa 30 mm und eine Länge von etwa 60 mm aufweist, ist zu Einführung in das Rektum des zu behandelnden Patienten vorgesehen, aus dem dann der Handgriff 3 des Handstückes 1 vorsteht. Das Handstück 1 ist über ein Verbindungskabel 4 mit dem in Fig. 3 veranschaulichten restlichen Elementen der Vorrichtung verbunden.

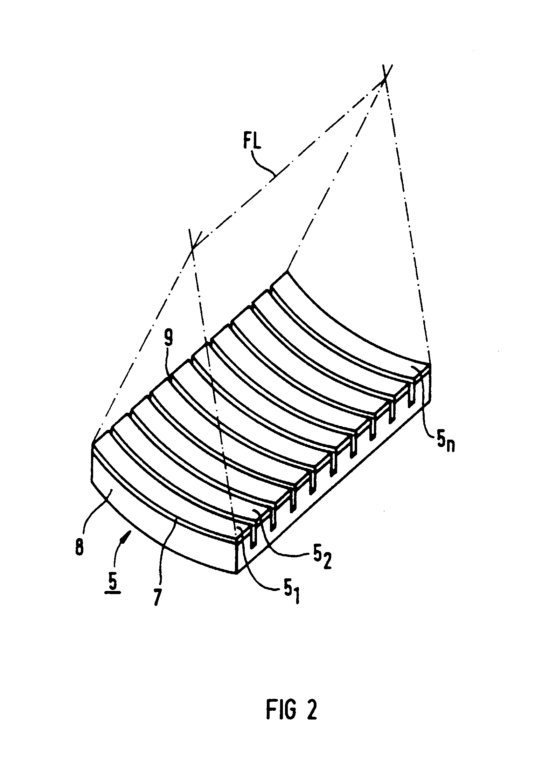

Das Handstück 1 enthält in seinem mit einem akustischen Ausbreitungsmedium, beispielsweise Wasser, gefällten Applikationsende als elektroakustischen Wandler einen in Fig. 2 näher dargestellten Ultraschallwandler 5. Im Bereich des Handgriffes 3 des Handstückes 1 ist ein Taster 6 angebracht, mittels dessen es dem behandelnden Arzt möglich ist, die Vorrichtung von Ortungs- auf Therapiebetrieb umzuschalten, wobei der Therapiebetrieb durch Drücken des Tasters 6 eingeschaltet wird, während bei unbetätigtem Taster 6 der Ortungsbetrieb eingeschaltet ist.

Im Ortungsbetrieb erzeugt der Ultraschallwandler 5 diagnostische akustische Wellen in Form von kurzen Ultraschallpulsen, deren Länge einige Halbperioden beträgt. Im Therapiebetrieb erzeugt der Ultraschallwandler 5 zusätzlich fokussierte therapeutische akustische Wellen in Form von Ultraschallwellen. Bei den therapeutischen Ultraschallwellen handelt es sich um Dauerschall oder um gepulsten Dauerschall der jeweils kurzzeitig zur Abstrahlung der diagnostischen Ultraschallwellen unterbrochen wird.

Der Ultraschallwandler 5 ist gemäß Fig. 2 als sogenanntes Linear Array aufgebaut, d.h. der Ultraschallwandler 5 ist in eine Anzahl von Ultraschallwandlerelementen 5₁, 5₂ usw. bis 5ₙ unterteilt. Die Unterteilung ist derart ausgeführt, daß es grundsätzlich Möglich ist, jedes der Ultraschallwandlerelemente 5₁ bis 5ₙ durch Zufuhr eines geeigneten elektrischen Signals einzeln zur Erzeugung von Ultraschallwellen anzutreiben. Ebenso ist es möglich, in der im Ortungsbetrieb erforderlichen Weise, die durch den Empfang im Körper des zu behandelnden Lebewesens reflektierter Anteile der diagnostischen Ultraschallwellen entstehenden elektrischen Signale für die einzelnen Ultraschallwandlerelemente 5₁ bis 5ₙ separat abzugreifen.

Der Übersichtlichkeit halber ist der in Fig. 2 dargestellte Ultraschallwandler nur in einige wenige, nämlich 10, Ultraschallwandlerelemente unterteilt. In der Praxis ist der Ultraschallwandler 5 in beispielsweise 128, 192 oder 256 Ultraschallwandlerelemente unterteilt. Der Ultraschallwandler ist in an sich bekannter Weise derart aufgebaut, daß das eigentliche piezoelektrische Material 7 in Form einer Schicht konstanter Dicke auf einem Tragkörper 8 geeigneter akustischer Impedanz ebenfalls konstanter Dicke aufgebracht ist. Dabei erfolgt die Verbindung der Schicht 7 piezoelektrischen Materials mit dem Tragkörper 8 in nicht dargestellter Weise durch eine metallische Schicht, deren Dicke klein im Verhältnis zu der der Schicht 7 ist. Die von dem Tragkörper 8 abgewandte Fläche der Schicht 7 ist ebenfalls mit einer dünnen, nicht dargestellten metallischen Schicht versehen. Die genannten metallischen Schichten dienen als Elektroden zur elektronischen Kontaktierung der Ultraschallwandlerelemente 5₁ bis 5ₙ.

Um Ultraschallwandlerelemente 5₁ bis 5ₙ zu erhalten, die unabhängig voneinander ansteuerbar sind und deren Ausgangssignale unabhängig voneinander abgefragt werden können, ist die mit dem Tragkörper 8 verbundene piezoelektrische Schicht 7 durch quer zur Längsachse des Ultraschallwandlers 5 verlaufende schmale Einschnitte, einer davon ist in Fig. 2 mit 9 bezeichnet, in die einzelnen Ultraschallwandlerelemente 5₁ bis 5ₙ unterteilt. Um die Ultraschallwandlerelemente 5₁ bis 5ₙ mechanisch voneinander zu entkoppeln, weisen die Einschnitte 9 eine Tiefe auf, die deutlich größer als die Dicke der piezoelektrischen Schicht 7 ist.

Bei geeigneter Ansteuerung der einzelnen Ultraschallwandlerelemente 5₁ bis 5ₙ ist es möglich, die von dem Ultraschallwandler 5 abgestrahlten Ultraschallwellen auf eine Fokuszone zu fokussieren und die Fokuszone der Ultraschallwellen zu verlagern. Bekanntermaßen ist auf diesem Wege eine Fokussierung bzw. die Ausführung einer Abtastbewegung nur in Richtung der Längsachse des Ultraschallwandlers 5 bzw. des linear array möglich. Um eine Fokussierung auch quer dazu zu erreichen, ist der Ultraschallwandler 5 in der in Fig. 2 angedeuteten, aus der US 41 59 462 im Zusammenhang mit diagnostischen Ultraschallwandlern an sich bekannten Weise um eine parallel zu seiner Längsachse verlaufende Achse zylindrisch gekrümmt, so daß sich bei gleichzeitiger Ansteuerung aller Ultraschallwandlerelemente 5₁ bis 5ₙ eine Fokussierung der Ultraschallwellen auf einen in Fig. 2 mit FL bezeichneten Linienfokus ergibt, der parallel zur Längsachse des Ultraschallwandlers 5 bzw. des linear array verläuft. Bei Ansteuerung des Ultraschallwandlers 5 mittels eines an sich bekannten diagnostischen Ultraschallgerätes 13 (siehe Fig. 3) nach Art eines phased array ist beispielsweise eine rechteckförmige Körperschicht des zu behandelnden Lebewesens zu Ortungszwecken abtastbar. Ein entsprechendes Rechteck ist in Fig. 1 angedeutet und mit R bezeichnet. Im Therapiebetrieb kann das in Fig. 1 mit F bezeichnete Zentrum der jeweils eingestellten Fokuszone in der Mittelebene der rechteckförmigen Schicht verlagert werden.

Wie die Ansteuerung der Ultraschallwandlerelemente 5₁ bis 5ₙ erfolgt, wird im folgenden anhand der Fig. 3 näher beschrieben. Dort sind von den Ultraschallwandlerelementen 5₁ bis 5ₙ exemplarisch die Ultraschallwandlerelemente 5₁ bis 5₃ und sₙ₁ bis 5ₙ dargestellt. Diese sind jeweils über eine Leitung 4₁ bis 4ₙ des Verbindungskabels 4 mit einem Schalter 10₁ bis 10ₙ verbunden. Die Schalter 10₁ bis 10ₙ sind Bestandteile einer insgesamt mit 11 bezeichneten Steuer- und Bilderzeugungselektronik. Die Schalter 10₁ bis 10ₙ, bei denen es sich vorzugsweise um elektronische Schalter handelt, werden von einer Ansteuerstufe 12 derart betätigt, daß sämtliche Schalter 10₁ bis 10ₙ jeweils die gleiche Schaltstellung einnehmen. Dies ist in Fig. 3 dadurch veranschaulicht, daß die Schalter 10₁ bis 10ₙ durch eine strichlierte Linie miteinander verbunden sind.

Wenn die Schalter 10₁ bis 10ₙ ihre in Fig. 3 nicht dargestellte Schaltstellung einnehmen, die dem Ortungsbetrieb entspricht, sind Ultraschallwandlerelemente 5₁ bis 5ₙ mit einem diagnostischen Ultraschallgerät 13 verbunden, das mit dem Ultraschallwandler 5 in an sich bekannter Weise derart zusammen wirkt, daß durch einen linear scan ein Bild der die Längsachse des Ultraschallwandler 5 und den Linienfokus FL enthaltenden Körperschicht eines zu behandelnden Patienten erzeugt und auf einem Monitor 17 angezeigt wird.

An das Ultraschallgerät 13 ist ein Joystick 18 angeschlossen, mittels dessen es möglich ist, eine in das auf dem Monitor 17 dargestellte Ultraschallbild eingeblendete Marke F' zu verschieben. Über einen Leitungsbus 19 gelangen entsprechend Daten bzw. Signale zu einer Steuereinheit 20, die Bestandteil der Steuer- und Bilderzeugungselektronik 11 ist, die dafür sorgt, daß die von den Ultraschallwandlerelementen 5₁ bis 5ₙ im Therapiebetrieb ausgehenden therapeutischen Ultraschallwellen auf eine Fokuszone fokussiert sind, deren Zentrum F im Körper des zu behandelnden Lebewesens an der Stelle liegt, die der mittels der Marke F' in dem Ultraschallbild markierten Stelle entspricht.

Bei den therapeutischen Ultraschallwellen handelt es sich um Dauerschall oder um gepulsten Dauerschall. Die therapeutischen Ultraschallwellen werden im Therapiebetrieb, der wie bereits erwähnt durch Betätigung des Tasters 6 eingeschaltet wird, periodisch kurzzeitig unterbrochen, um auch während des Therapiebetriebes das Ultraschallbild zu aktualisieren. Zu diesem Zweck wirkt eine zu der Steuer- und Bilderzeugungselektronik 11 gehörige Steuereinheit 20 auf die Ansteuerstufe 12 ein und bringt die Schalter 10₁ bis 10ₙ in die für die Erzeugung eines Ultraschallbildes erforderliche Zeit in die ein Ortungsbetrieb entsprechende Stellung. Danach kehren die Schalter bis zur Anfertigung des darauffolgenden Ultraschallbildes in ihre dem Therapiebetrieb entsprechende Schaltstellung zurück. Während die Ultraschallbilder im Ortungsbetrieb mit einer Folgefrequenz von z.B. 25 Hz erzeugt werden, liegt die Folgefrequenz im Therapiebetrieb beispielsweise bei 0,1 bis 1 Hz. Da die zur Erzeugung eines Ultraschallscans erforderliche Zeit sehr kurz ist (deutlich kürzer als 1 Millisekunde), kann die Pulsdauer der therapeutischen Ultraschallwellen für vernachlässigbar kurze Pausen unterbrochen werden, um pulsartige diagnostische Ultraschallwellen zu erzeugen. Die Pulsdauer der therapeutischen Ultraschallwellen beträgt maximal ca. 1 bis 10 Sekunden. Kürzere Pulsdauern können eingestellt werden.

Nehmen die Schalter 10₁ bis 10ₙ dagegen ihre in Fig. 3 dargestellte Schaltstellung ein, die dem Therapiebetrieb entspricht, sind die Ultraschallwandlerelemente 5₁ bis 5ₙ des Ultraschallwandlers 5 mit einer Einrichtung zur Ansteuerung des Ultraschallwandlers 5 verbunden. Diese enthält für jedes der Ultraschallwandlerelemente 5₁ bis 5ₙ einen Speicher 16₁ bis 16ₙ, deren Datenausgängen Dₒᵤₜ nachgeschaltete Treiberstufen 15₁ bis 15ₙ, und zwischen letztere und die Ultraschallwandlerelemente 5₁ bis 5ₙ geschaltete, Signalumformmittel bildende LC-Netzwerke 14₁ bis 14ₙ. Bei den Speichern handelt es sich um Schreib/Lese-Speicher (RAM). Auch die Steuer- und Bilderzeugungselektronik 11 ist, wenigstens zum Teil, Bestandteil der Einrichtung zur Ansteuerung des Ultraschallwandlers 5.

Die Adreßeingänge ADR und die Dateneingänge Dᵢₙ der Speicher 16₁ bis 16ₙ sind über einen Adreßbus 21 und eine Datenleitung 22 mit der Steuer- und Bilderzeugungselektronik 11 verbunden, die übrigens eine der Bedienung der Vorrichtung dienende Tastatur 23 enthält.

Sobald eine gewünschte Position der Fokuszone mittels des Joysticks 18 angewählt ist, schaltet die Steuer- und Bilderzeugungselektronik 11 die Speicher 16₁ bis 16ₙ über eine zu deren WE(Write Enable)-Eingängen führende Leitung 24 in den Schreibmodus und schreibt in die Speicher 16₁ bis 16ₙ der gewünschten Position des Fokus entsprechende bit-Muster ein. Da die Speicher 16₁ bis 16ₙ während dieses Schreibvorganges aufeinanderfolgend adressiert werden und jeweils nur eine Speichertiefe von einem bit aufweisen, genügt eine einzige Datenleitung 22, die die Dateneingänge sämtlicher Speicher 16₁ bis 16ₙ mit der Steuer- und Bilderzeugungselektronik 11 verbindet. Der beschriebene Schreibvorgang erfolgt sequentiell in dem Sinne, daß jeweils nur einer der Speicher 16₁ bis 16ₙ durch die entsprechende, zu einem Leitungsbus 25 gehörige Freigabeleitung 25₁ bis 25ₙ freigegeben ist. Die Freigabeleitungen 25₁ bis 25ₙ sind an die CS(Chip Select)-Eingänge der Speicher 16₁ bis 16ₙ angeschlossen.

Wenn in jeden der Speicher 16₁ bis 16ₙ das der gewünschten Position des Fokus entsprechende bit-Muster eingeschrieben ist und die Vorrichtung durch Betätigung des Tasters 6 in den Therapiebetrieb geschaltet wird, schaltet die Steuer- und Bilderzeugungselektronik 11 alle Speicher 16₁ bis 16ₙ über die Leitung 24 in den Lesemodus und adressiert diese parallel und zyklisch so daß an den Datenausgängen der Speicher 16₁ bis 16ₙ Rechtecksignale auftreten, die entsprechend der gewünschten Position des Fokus zeitlich zueinander versetzt sind. Unter paralleler Adressierung soll hier verstanden werden, daß zu einem gegebenen Zeitpunkt bei sämtlichen Speichern 16₁ bis 16ₙ jeweils die gleiche Speicherzelle adressiert wird. Unter zyklischer Adressierung soll verstanden werden, daß auf- und absteigend aufeinanderfolgend sämtliche Speicherzellen der Speicher 16₁ bis 16ₙ adressiert werden und dieser Vorgang dann immer wieder wiederholt wird, und zwar so lange, wie die Vorrichtung in den Therapiemodus geschaltet ist. Dabei sind wie erwähnt von Zeit zu Zeit kurze Unterbrechungen zur Anfertigung aktualisierter Ultraschallbilder vorgesehen sein. Es versteht sich, daß in den bit-Mustern berücksichtigt sein muß, ob die zyklische Adressierung auf- und oder absteigend erfolgt.

Die an den Datenausgängen der Speicher 16₁ bis 16ₙ auftretenden Rechtecksignale haben eine Frequenz, die dem Quotienten aus der Taktfrequenz, mit der die zyklische Adressierung der Speicher 16₁ bis 16ₙ erfolgt, und aus der Speicherlänge entspricht, im Falle des beschriebenen Ausführungsbeispieles 40 MHz bzw. 16 bit. Damit ergibt sich im Falle des beschriebenen Ausführungsbeispieles eine Frequenz der Rechtecksignale von 2,5 MHz.

Die Rechtecksignale werden nach Durchlaufen der Treiberstufen 15₁ bis 15ₙ durch die LC-Netzwerke in Sinussignale umgeformt, deren Frequenz der der Rechtecksignale entspricht.

Die Ultraschallwandlerelemente 5₁ bis 5ₙ des Ultraschallwandlers 5 werden also nach Art eines an sich bekannten phased array mit Sinussignalen angesteuert, die derart relativ zueinander phasenversetzt sind, daß die mittels des Ultraschallwandlers 5 erzeugten Ultraschallwellen auf einen Fokus fokussiert sind, der die jeweils gewünschte Position einnimmt.
Die zum Einschreiben der bit-Muster in die Speicher 16₁ bis 16ₙ bzw. zum Auslesen der bit-Muster aus den Speichern 16₁ bis 16ₙ erforderlichen Adreßsignale und Steuersignale erzeugt ein Adreß- und Taktgenerator 26, an den der Adreßbus 21, die Datenleitung 22, die Leitung 24 und der Leitungsbus 25 angeschlossen sind. Der Adreß- und Taktgenerator 26 wird von der Steuereinheit 20 gesteuert, mit der er über einen Leitungsbus 19 verbunden ist.

In einer ersten mittels der Tastatur 23 einstellbaren Betriebsart errechnet die Steuereinheit 20 die zu der jeweils mittels des Joysticks 18 angewählten Position des Fokus gehörigen bit-Muster. Die entsprechenden Daten gelangen dann über den Leitungsbus 19 zu der Steuereinheit 20 und von dort in die Speicher 16₁ bis 16ₙ.

In einer zweiten ebenfalls mittels der Tastatur 23 anwählbaren Betriebsweise entnimmt der Adreß- und Taktgenerator 26 die zu einer mittels des Joysticks 18 angewählten Position des Fokus gehörigen bit-Muster einem vorzugsweise als nicht flüchtiger Speicher ausgeführten Datenspeicher, im Falle des beschriebenen Ausführungsbeispiels einem EPROM 28, und schreibt sie in die Speicher 16₁ bis 16ₙ ein. Das EPROM 28 enthält die bit-Muster für eine Anzahl von matrixartig in einer Ebene angeordneter Positionen des Fokus, wobei die Ebene in der Körperschicht enthalten ist, die in dem mittels des Ultraschallgerätes 13 erzeugten Ultraschallbild abgebildet ist.

Die matrixartige Anordnung der Positionen des Fokus, bezüglich derer in dem EPROM 28 bit-Muster gespeichert sind, ist unter Berücksichtigung der Abmessungen des Fokus (in der Praxis ergibt sich kein punktförmiger Fokus, sondern eine Fokuszone) derart gewählt, daß ein Körperbereich des Patienten wenigstens im wesentlichen lückenlos behandelt werden kann. Als Ausdehnung des Fokus wird in vorteilhafter Weise die sogenannte -3db-Zone angenommen, also derjenige Bereich, innerhalb dessen der Schalldruck der Ultraschallwellen wenigstens 50% des auftretenden Spitzenschalldruckes beträgt.

Wird mittels des Joysticks 18 eine Position des Fokus angewählt, bezüglich derer keine bit-Muster in dem EPROM 28 gespeichert sind, übertragen die Steuereinheit 20 und der Adreß- und Taktgenerator 26 diejenigen bit-Muster aus dem EPROM 28 in die Speicher 16₁ bis 16ₙ, die der am nächsten bei der angewählten Position des Fokus liegenden Position entsprechen bezüglich derer bit-Muster in dem EPROM 28 gespeichert sind.

Die Treiberstufen 15₁ bis 15ₙ sind über eine Versorgungsleitung 29 mit einer zu der Steuer- und Bilderzeugungselektronik 11 gehörigen Stromversorgung 30 verbunden. Die Stromversorgung 30 ihrerseits ist über den Leitungsbus 19 mit der Steuereinheit 20 verbunden und derart ausgebildet, daß ihre Ausgangsspannung einstellbar ist. Dabei ist im Falle des beschriebenen Ausführungsbeispieles die Anordnung derart getroffen, daß mittels Eingabe über die Tastatur 23 die Ausgangsspannung der Stromversorgung 30 einstellbar ist. Die Treiberstufen 15₁ bis 15ₙ wiederum sind derart ausgebildet, daß die Amplitude der an ihren Ausgängen vorliegenden Rechtecksignale der über die Versorgungsleitung 29 zugeführten Versorgungsspannung proportional ist. Es besteht somit die Möglichkeit, die Amplitude der mittels des Ultraschallwandlers 5 erzeugten therapeutischen Ultraschallwellen dem jeweiligen Behandlungsfall anzupassen.

Da dies konstruktiv am einfachsten ist, werden im Falle des beschriebenen Ausführungsbeispieles sämtliche Treiberstufen 15₁ bis 15ₙ mit der gleichen Spannung versorgt. Um die Bildung von Nebenfoki zu verhindern, ist es jedoch zweckmäßig, wenn die im Bereich des Randes des Ultraschallwandlers 5 liegenden Ultraschallwandlerelemente, z.B. die Ultraschallwandlerelemente 5₁ und 5₂ sowie 5ₙ₋₁ und 5ₙ, Ultraschallwellen verminderter Intensität abstrahlen.

Dies wird im Falle des beschriebenen Ausführungsbeispieles dadurch erreicht, daß die Steuereinheit 20 in die zu im Bereich des Randes des Ultraschallwandlers 5 liegenden Ultraschallwandlerelementen gehörigen Speicher z.B. 16₁ und 16₂ bzw. 16ₙ₋₁ und 16ₙ von den übrigen Speichern derart abweichend bit-Muster einschreibt, daß die entsprechenden Ultraschallwellen eine verringernde Intensität aufweisen.

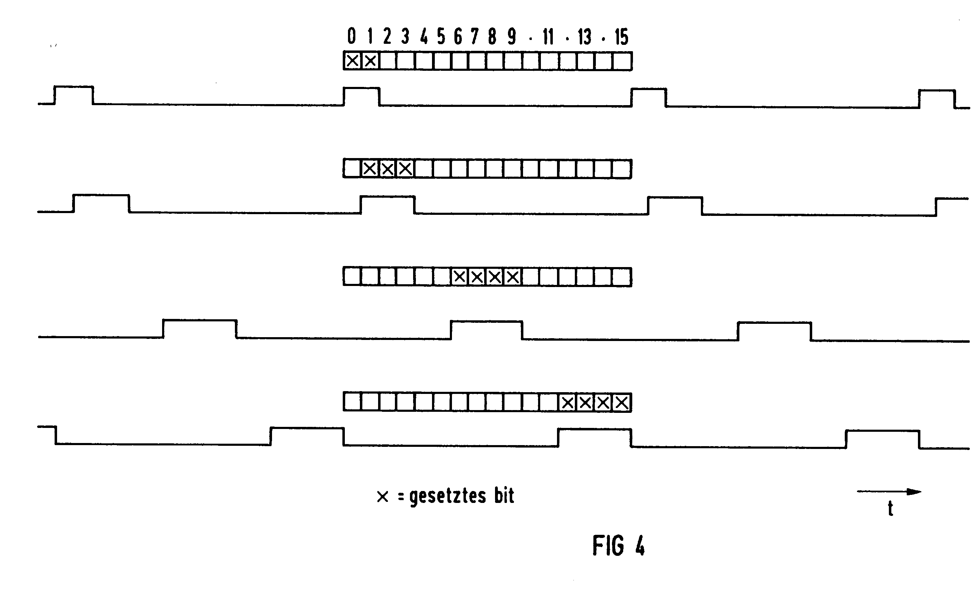

Wie aus der Fig. 4 ersichtlich ist, die für einige Ultraschallwandlerelemente (von oben nach unten: Ultraschallwandlerelemente 5₁ und 5ₙ, Ultraschallwandlerelemente 5₂ und 5ₙ₋₁, ein zwischen Mitte und Rand liegendes Ultraschallwandlerelement, ein mittleres Ultraschallwandlerelement) die in den entsprechenden Speichern gespeicherten bit-Muster und jeweils darunter die an den Datenausgängen des entsprechenden Speichers auftretenden Rechtecksignale zeigt, sind im Fälle der vom Rand des Ultraschallwandlers entfernten Ultraschallwandlerelemente jeweils vier aufeinanderfolgende bits gesetzt, während im Falle der randseitigen Ultraschallwandlerelemente eine verringerte Anzahl von aufeinanderfolgenden bits gesetzt sind. Dies hat zur Folge, daß nach der Wandlung der an den Ausgängen der Speicher auftretenden Rechtecksignale in sinusförmige Signale die zu den randseitigen Ultraschallwandlerelementen gehörigen Sinussignale einer verringerte Amplitude aufweisen.

Um im Therapiebetrieb im Interesse der Sicherheit des Patienten ausschließen zu können, daß therapeutische Ultraschallwellen zu hoher Amplitude erzeugt werden, ist eine Überwachungsschaltung 31 vorgesehen, die über eine Schematisch angedeutete Meßleitung 32 und ebenfalls schematisch angedeutete Meßleitungsbusse 33 und 34 die am Ausgang der Stromversorgung 30, den Ausgängen der Treiberschaltungen 15₁ bis 15ₙ und den Ausgängen der LC-Netzwerke 14₁ bis 14ₙ vorliegenden Spannungen sowie die an diesen stellen auftretenden Ströme mißt und mit entsprechenden Schwellwerten vergleicht. Wird einer der Schwellwerte überschritten, teilt die Überwachungsschaltung 31 dies der Steuereinheit 20 mit, mit der sie über den Leitungsbus 19 verbunden ist. Die Steuereinheit 20 senkt dann die von der Stromversorgung 30 gelieferte Versorgungsspannung so weit ab, daß eine Schwellwertüberschreitung nicht mehr vorliegt.

Zur Durchführung einer Behandlung wird derart vorgegangen, daß das Handstück 1 mit seinem Applikationsende 2 in das Rektum des Patienten eingeführt wird. Dabei unterbleibt zunächst die Betätigung des Tasters 6. Das Gerät befindet sich somit im Ortungsbetrieb . Das Handstück wird nun so ausgerichtet, daß das zu behandelnde Objekt im Ultraschallbild erscheint. Der behandelnde Arzt setzt nun mittels des Joysticks 18 die Marke F, auf die zu behandelnde Zone. Dann drückt er auf den Taster 6 am Handgriff 3, wodurch auf Therapiebetrieb geschaltet wird. Es werden dann therapeutische Ultraschallwellen abgestrahlt, deren Wirkzone im zu behandelnden Objekt eine solche Lage einnimmt, daß sich ihr Zentrum F an der der eingestellten Position der Marke F' entsprechenden Stelle befindet. Da während der Therapie in der zuvor beschriebenen Weise ständig kurzzeitig in den Ortungsbetrieb umgeschaltet wird, erhält der Anwender den Eindruck einer Echtzeit-Darstellung und wird ständig Über den therapeutischen Erfolg informiert. Da sich der Ultraschallwandler 5 in dem Handstück 1 befindet, ist es leicht möglich, während der Therapie die Wirkzone zu verlagern, da anhand der in das Ultraschallbild eingeblendeten Marke jederzeit die Lage der Wirkzone erkennbar ist.

Der Therapiebetrieb wird dadurch verlassen, daß der Taster 6 wieder losgelassen wird. Das Gerät läuft dann von selbst im Ortungsbetrieb weiter.

In einer weiteren Betriebsart besteht die Möglichkeit, mittels eines in Fig. 3 angedeuteten Lichtgriffels 35 (oder mittels einer Über den Joystick 18 verlagerbaren Marke) in dem auf dem Monitor angezeigten Ultraschallbild einen zu behandelnden Bereich zu umfahren. Die Position des Fokus wird dann von der Steuereinheit 20, die veranlaßt, daß die jeweils erforderlichen bit-Muster in die Speicher 161 bis 16n eingeschrieben werden, nach und nach so verlagert, bis der gesamte innerhalb des mit dem Lichtgriffel markierten Bereiches liegende Körperbereich mit therapeutischen akustischen Wellen behandelt ist. Es ist zweckmäßig, wenn in dieser Betriebsart Mittel zur Fixierung des Handstückes 1, beispielsweise ein nicht dargestelltes Stativ, vorgesehen sind, um die Gefahr von Verlagerungen des Handstückes 1 relativ zu dem Körper des Patienten zu minimieren.

Die Frequenz der im Therapiebetrieb erzeugten therapeutischer Ultraschallwellen ist vorzugsweise niedriger als die Frequenz der diagnostischen Ultraschallwellen ist, die im Ortungsbetrieb abgegeben werden. Es wird somit in vorteilhafter Weise bei der Erstellung der Ultraschallbilder eine hohe Ortsauflösung erzielt, so daß es möglich ist, die zu behandelnde Zone mit hoher Genauigkeit zu orten und die Wirkzone mit erhöhter Genauigkeit in der zu behandelnden Zone zu positionieren. Zugleich ist gewährleistet, daß die therapeutischen Ultraschallwellen nicht unnötigerweise gedämpft werden.

Insbesondere dann, wenn durch das Ultraschallgerät 13 die Möglichkeit gegeben ist zur Ortung nicht nur linear scans, sondern auch sector scans auszufahren, ist es zweckmäßig, die Breite b der Ultraschallwandlerelemente 5₁ bis 5ₙ so zu wählen, daß sie kleiner als die halbe Wellenlänge der diagnostischen Ultraschallwellen in dem jeweiligen akustischen Ausbreitungsmedium, d.h. dem in dem Handstück 1 enthaltenen Ausbreitungsmedium bzw. dem Körpergewebe des zu behandelnden Lebewesens, ist. Hierdurch ist sichergestellt, daß die Abstrahlung der diagnostischen Ultraschallwellen ungerichtet erfolgt, was Voraussetzung dafür ist, in der beschriebenen Weise eine sektorförmige Körperschicht eines zu behandelnden Lebewesens abtasten zu können.

Da dies zu einer zahl der Ultraschallwandlerelemente führen kann, die für die Erzeugung der therapeutischen Ultraschallwellen wegen deren größerer Wellenlänge an sich zu groß ist, kann vorgesehen sein, daß zur Erzeugung der therapeutischen Ultraschallwellen jeweils eine Anzahl von Ultraschallwandlerelementen zu einer Ultraschallwandlerelementengruppe zusammengefaßt ist. Im Falle des beschriebenen Ausführungsbeispiels kann dies dadurch geschehen, daß in die zu einer Ultraschallwandlerelementengruppe gehörigen Speicher jeweils das gleiche bit-Muster eingeschrieben wird.

Die Anzahl der jeweils zu einer Ultraschallwandlerelementengruppe gehörigen Ultraschallwandlerelemente würde dann dem Quotienten aus der Anzahl der insgesamt vorhandenen Ultraschallwandlerelementen und aus der Speicherlänge entsprechen. Es versteht sich, daß sichergestellt sein muß, daß die Gesamtbreite einer Ultraschallwandlerelementengruppe kleiner als die halbe Wellenlänge der therapeutischen Ultraschallwellen im jeweiligen akustischen Ausbreitungsmedium ist. Die Abstrahlung der therapeutischen Ultraschallwellen erfolgt dann ungerichtet, was Voraussetzung ist, um die Wirkzone in der beschriebenen Weise verlagern zu können.

Wenn vorstehend von einem Ultraschallwandlerelement die Rede ist, kann es sich hierbei tatsächlich um ein einziges Ultraschallwandlerelement im engeren Sinne handeln. Es besteht aber auch in aus der DE 43 02 538 C1 bekannter Weise die Möglichkeit, ein Ultraschallwandlerelement durch mehrere parallelgeschaltete Ultraschallwandler(teil)elemente zu bilden, welche über ein gemeinsames LC-Netzwerk und eine gemeinsamen Treiberstufe mit einem gemeinsamen Speicher in Verbindung stehen. Es können also beispielsweise 256 Ultraschallwandler(teil)elemente zu 16 Ultraschallwandlerelementen zusammengefaßt sein, von denen jedes 16 Ultraschallwandler(teil)elemente enthält. Es ist dann für jedes der aus jeweils 16 Ultraschallwandler(teil)elementen gebildeten Ultraschallwandlerelemente ein LC-Netzwerk, eine Treiberstufe und ein Speicher erforderlich. Auch hier gilt, daß die Anzahl der zu einem Ultraschallwandlerelement zusammengefaßten Ultraschallwandler(teil)elemente so gewählt sein muß, daß die Breite des resultierenden Ultraschallwandlerelementes kleiner als die halbe Wellenlänge der therapeutischen Ultraschallwellen im jeweiligen akustischen Ausbreitungsmedium ist.

Die Frequenz der therapeutischen Ultraschallwellen und die der diagnostischen Ultraschallwellen sind in aus der DE 43 02 538 C1 bekannter Weise derart gewählt, daß die Ultraschallwandlerelemente 5₁ bis 5ₙ des Ultraschallwandlers 5 sowohl im Therapie- als auch im Ortungsbetrieb unter Resonanzbedingungen arbeiten (Grundwelle/Oberwelle).

Bezüglich der für die Behandlung der benignen Prostata-Hyperplasie geeigneten Frequenzen im Therapie- bzw. Ortungsbetrieb wird auf die bereits genannte DE 43 02 538 C1 verwiesen.

Auch bezüglich der Breite b und der Dicke der Ultraschallwandlerelemente 5₁ bis 5ₙ wird auf die bereits genannte ,DE 43 02 538 C1 verwiesen.

Im Falle des beschriebenen Ausführungsbeispieles besteht die Möglichkeit, die in die Speicher 16₁ bis 16ₙ einzuschreibenden bit-Muster wahlweise dem EPROM 28 zu entnehmen oder von der Steuereinheit 20 errechnen zu lassen. Es sind aber auch Ausführungsformen möglich, die nur von der einen oder anderen Möglichkeit Gebrauch machen.

Bei dem Ultraschallwandler 5 in Falle des beschriebenen Ausführungsbeispieles handelt es sich um ein eindimensionales Array von Ultraschallwandlerelementen 5₁ bis 5ₙ. Demnach läßt sich der Fokus der erzeugten Ultraschllwellen zweidimensional und zwar in einer Ebene, verlagern. Es besteht auch die Möglichkeit, als Ultraschallwandler ein zweidimensionales Array von Ultraschallwandlerelementen zu verwenden. Der Fokus der erzeugten therapeutischen Ultraschallwellen kann dann dreidimensional verlagert werden. Ein zweidimensionales Array von Ultraschallwandlerelementen kann beispielsweise eine Vielzahl von matrixartigen in einer Ebene angeordneten Ultraschallwandlerelementen aufweisen. Es besteht auch die Möglichkeit, Antriebsmittel zur Verlagerung des Ultraschallwandlers 5 (z.B. innerhalb des Handstückes 1) relativ zu dem Körper des Patienten vorzusehen, um eine Verlagerung des Fokus zu ermöglichen.

Das vorstehend beschriebene Ausführungsbeispiel betrifft eine Vorrichtung, die rektal appliziert, also teilinvasiv angewendet wird und zur Behandlung der benignen Prostatahyperplasie vorgesehen ist. Es können aber auch andere Vorrichtungen, die extrakorporal, d.h. nicht invasiv, appliziert werden und/oder zur Behandlung anderer Leiden dienen, erfindungsgemäß ausgebildet werden.

Im Falle des beschriebenen Ausführungsbeispieles sind zwischen die Datenausgänge der Speicher und die Ultraschallwandlerelemente LC-Netzwerke und Treiberstufen geschaltet. Diese können zugunsten einer direkten Verbindung der Datenausgänge der Speicher und der Ultraschallwandlerelemente entfallen, wenn die Datenausgänge der Speicher einen ausreichend hohen Strom liefern können. Die an den Datenausgänge der Speicher zur Verfügung stehend Signale werden dann den Ultraschallwandlerelementen unmittelbar zur Ultraschall-Erzeugung zugeführt.

Unter einem phased array ist übrigens eine durch zeitlich verzögerte Ansteuerung elektronisch fokussierbare Anordnung einer Vielzahl von Ultraschallwandlerelementen zu verstehen. Ein linear array ist eine lineare Anordnung einer Vielzahl von Ultraschallwandlerelementen. Unter einem scan ist eine beispielsweise lineare (linear scan) oder sektorförmige (sector scan) Abtastung mittels eines Ultraschall-Strahles zu verstehen.

## Patentansprüche

1. Vorrichtung zur Behandlung eines Objektes mit fokussierten Ultraschallwellen, die einen aus einer Vielzahl von Ultraschallwandlerelementen (5₁ bis 5ₙ) zusammengesetzten Ultraschallwandler (5) aufweist, gekennzeichnet durch eine Einrichtung zur Ansteuerung des Ultraschallwandlers (5), welche für jedes Ultraschallwandlerelement (5₁ bis 5ₙ) einen Speicher (16₁ bis 16ₙ) aufweist, in dem im Betrieb ein einer gewünschten Position des Fokus (F) der Ultraschallwellen entsprechendes bit-Muster gespeichert ist, und eine Steuereinheit (20), die die einzelnen Speicher (16₁ bis 16ₙ) parallel derart zyklisch adressiert, daß an den Datenausgängen der Speicher (16₁ bis 16ₙ) entsprechend der gewünschten Position des Fokus (F) zeitlich zueinander versetzte elektrische Signale auftreten, die dem jeweiligen Ultraschallwandlerelement (5₁ bis 5ₙ) zugeführt sind

2. Vorrichtung nach Anspruch 1, welche als Speicher (16₁ bis 16ₙ) Schreib-/Lesespeicher enthält, in die die Steuereinheit (20) die der gewünschten Position des Fokus (F) entsprechenden bit-Muster einschreibt.

3. Vorrichtung nach Anspruch 1 oder 2, deren Steuereinheit (20) die einer gewünschten Position des Fokus (F) entsprechenden bit-Muster errechnet und in den Speicher (16₁ bis 16ₙ) einschreibt.

4. Vorrichtung nach einem der Ansprüche 1 bis 3, deren Steuereinheit (20) einen Datenspeicher (28) aufweist, in dem für unterschiedliche Positionen des Fokus (F) bit-Muster gespeichert sind, wobei die Steuereinheit (20) die der jeweils gewünschten Position des Fokus (F) entsprechenden bit-Musters in die Speicher (16₁ bis 16ₙ) einschreibt.

5. Vorrichtung nach Anspruch 4, in deren Datenspeicher (28) bit-Muster für unterschiedlichen Positionen des Fokus (F) gespeichert sind, die zwei- oder dreidimensional matrixartig angeordnet.

6. Vorrichtung nach einem der Ansprüche 1 bis 5, deren Steuereinheit (20) Eingabemittel (18, 35) für eine gewünschte Position des Fokus (F) zugeordnet sind.

7. Vorrichtung nach Anspruch 6, welche eine Ortungseinrichtung aufweist, mittels derer ein Bild zumindest eines Bereichs des zu behandelnden Objektes erzeugbar ist, und deren Eingabemittel (18, 35) derart ausgebildet sind, daß die jeweils gewünschte Position des Fokus (F) in dem Bild anwählbar ist.

8. Vorrichtung nach Anspruch 7, welche als Ortungseinrichtung eine Ultraschall-Ortungseinrichtung (5, 13, 17) aufweist.

9. Vorrichtung nach Anspruch 8, deren Ultraschall-Ortungseinrichtung (5, 13, 17) ein Schnittbild zumindest eines Bereichs des zu behandelnden Objektes erzeugt, wobei die in dem Datenspeicher (28) gespeicherten unterschiedlichen Positionen des Fokus (F) in einer Ebene liegen, die in dem in dem Schnittbild abgebildeten Bereich des zu behandelnden Objektes enthalten ist.

10. Vorrichtung nach Anspruch 8 oder 9, deren Ultraschallwandler (5) Bestandteil der Ultraschall-Ortungseinrichtung (5, 13, 17) ist

11. Vorrichtung nach einem der Ansprüche 1 bis 10, deren Ultraschallwandler (5) eine lineare Anordnung (linear array) von Ultraschallwandlerelementen (5₁ bis 5ₙ) enthält.

12. Vorrichtung nach den Ansprüchen 10 und 11, mittels deren Ultraschallwandler (5) das zu behandelnde Objekt zur Erzeugung eines Ultraschall-Schnittbildes in einem linearen Abtastvorgang (linear scan) abgetastet wird.

13. Vorrichtung nach einem der Ansprüche 1 bis 12, welche zwischen die Datenausgänge der Speicher (16₁ bis 16ₙ) und die entsprechenden Ultraschallwandlerelemente (5₁ bis 5ₙ) geschaltete Signalumformungsmittel aufweist, die die an den Datenausgängen der Speicher (16₁ bis 16ₙ) auftretenden rechteckartigen Signale in wenigstens im wesentlichen sinusförmige Signale umwandeln.

14. Vorrichtung nach Anspruch 13, deren Signalumformungsmittel LC-Netzwerke (14₁ bis 14ₙ) enthalten.

15. Vorrichtung nach einem der Ansprüche 1 bis 14, deren Speicher (16₁ bis 16ₙ) jeweils eine Speichertiefe von 1 bit aufweisen.

16. Vorrichtung nach einem der Ansprüche 1 bis 15, deren Speicher (16₁ bis 16ₙ) jeweils eine Speicherlänge von 16 bit aufweisen.

17. Vorrichtung nach einem der Ansprüche 1 bis 16, bei der die Taktfrequenz, mit der die Steuereinheit (20) die Speicher (16₁ bis 16ₙ) adressiert, gleich der mit der Speicherlänge multiplizierten Frequenz der Ultraschallwellen ist.

18. Vorrichtung nach einem der Ansprüche 1 bis 17, welche zwischen die Datenausgänge der Speicher (16₁ bis 16ₙ) und die entsprechenden Ultraschallwandlerelemente (5₁ bis 5ₙ) geschaltete Treiberstufen (15₁ bis 15ₙ) aufweist.

19. Vorrichtung nach Anspruch 18, deren Treiberstufen (15₁ bis 15ₙ) mit einer Stromversorgungseinheit (30) verbunden sind, deren Ausgangsspannung mittels der Steuereinheit (20) einstellbar ist.

20. Vorrichtung nach einem der Ansprüche 1 bis 19, welche Überwachungsmittel aufweist, die Spannung und/oder Strom an den Ausgängen der Treiberstufen (15₁ bis 15ₙ) und/oder an den Ausgängen der Signalumformungsmittel und/oder am Ausgang der Stromversorgung (30) messen und mit einem entsprechenden Referenzwert bzw. entsprechenden Referenzwerten vergleichen.

21. Vorrichtung nach einem der Ansprüche 1 bis 20, deren Steuereinheit (20) in die zu im Bereich des Randes des Ultraschallwandlers (5) liegenden Ultraschallwandlerelementen (5₁ und 5₂, 5ₙ₋₁ und 5ₙ) gehörige Speicher (16₁ und 16₂, 16ₙ₋₁ und 16ₙ) von den übrigen Speichern derart abweichende bit-Muster einschreibt, daß die von dem im Bereich des Randes des Ultraschallwandlers (5) liegenden Ultraschallwandlerelementen (5₁ und 5₂, 5ₙ₋₁ und 5ₙ) ausgehenden Ultraschallwellen eine im Vergleich zu den von den übrigen Ultraschallwandlerelementen ausgehenden Ultraschallwellen verringerte Intensität aufweisen.

22. Vorrichtung nach Anspruch 21, deren Steuereinheit (20) in die zu nicht im Bereich des Randes des Ultraschallwandlers (5) liegenden Ultraschallwandlerelementen gehörigen Speicher bit-Muster einschreibt, die jeweils eine Folge unmittelbar aufeinanderfolgender gesetzter bits enthalten, und in die zu im Bereich des Randes des Ultraschallwandlers (5) liegenden Ultraschallwandlerelementen (5₁ und 5₂, 5ₙ₋₁ und 5ₙ) gehörigen Speicher (16₁ und 16₂, 16ₙ₋₁ und 16ₙ) bit-Muster einschreibt, die eine verringerte Anzahl von aufeinanderfolgenden gesetzten bits enthalten.

## Claims

1. Device for treating a subject with focussed ultrasonic waves, which has an ultrasonic transducer (5) composed of a number of ultrasonic transducer elements (5₁ to 5ₙ), **characterised by** a device for driving the ultrasonic transducer (5), which has a memory (16₁ to 16ₙ) for each ultrasonic transducer element (5₁ to 5ₙ) in which a bit pattern is stored, corresponding to a desired position of the focus (F) of the ultrasonic waves during operation, and a control unit (20) which cyclically addresses the individual memories (16₁ to 16ₙ) in parallel so that electrical signals, offset in time relative to each other according to the desired position of the focus (F), appear at the data outputs of the memories (16₁ to 16ₙ), and said electrical signals are fed to the respective ultrasonic transducer element (5₁ to 5ₙ)

2. Device according to Claim 1, which as a memory (16₁ to 16ₙ), contains a read/write memory into which the control unit (20) writes the bit pattern corresponding to the desired position of the focus (F).

3. Device according to Claim 1 or 2, whose control unit (20) calculates the bit pattern corresponding to a desired position of the focus (F) and writes said bit pattern into the memory (16₁ to 16ₙ).

4. Device according to one of Claims 1 to 3, whose control unit (20) has a data memory (28) in which bit patterns are stored for different positions of the focus (F), the control unit (20) writing the bit pattern corresponding to the respective desired position of the focus (F) into the memory (16₁ to 16ₙ).

5. Device according to Claim 4, in whose data memory (28) bit patterns are stored for different positions of the focus (F) and are arranged in a two-dimensional or three-dimensional matrix.

6. Device according to one of Claims 1 to 5, to whose control unit (20) input means (18, 35) are allocated for a desired position of the focus (F).

7. Device according to Claim 6, which has a locating device by which an image of at least one region of the subject to be treated can be produced, and whose input means (18, 35) are constructed so that the respective desired position of the focus (F) can be selected in said image.

8. Device according to Claim 7, which has an ultrasonic locating device (5, 13, 17) as said locating device.

9. Device according to Claim 8, whose ultrasonic locating device (5, 13, 17) produces a sectional view of at least one region of the subject to be treated, the different positions of the focus (F) stored in the data memory (28) lying in a plane that is included in the region of the subject to be treated and displayed in said sectional view.

10. Device according to Claim 8 or 9, whose ultrasonic transducer (5) is a constituent part of the ultrasonic locating device (5, 13, 17).

11. Device according to one of Claims 1 to 10, whose ultrasonic transducer (5) contains a linear array of ultrasonic transducer elements (5₁ to 5ₙ).

12. Device according to Claims 10 and 11, by means of whose ultrasonic transducer (5) the subject to be treated is scanned in a linear scan in order to produce an ultrasonic tomogram.

13. Device according to one of Claims 1 to 12, which has signal conversion means connected between the data output of the memories (16₁ to 16ₙ) and the corresponding ultrasonic transducer elements (5₁ to 5ₙ), which convert the square wave signals appearing at the data outputs of the memories (16₁ to 16ₙ) into at least substantially sinusoidal signals.

14. Device according to Claim 13, whose signal conversion means include LC networks (14₁ to 14ₙ).

15. Device according to one of Claims 1 to 14, each of whose memories (16₁ to 16ₙ) has a memory depth of 1 bit.

16. Device according to one of Claims 1 to 15, each of whose memories (16₁ to 16ₙ) has a memory length of 16 bits.

17. Device according to one of Claims 1 to 16, in which the clock frequency with which the control unit (20) addresses the memories (16₁to 16ₙ) is equal to the frequency of the ultrasonic waves multiplied by said memory length.

18. Device according to one of Claims 1 to 17, which has driver stages (15₁ to 15ₙ) connected between the data outputs of the memories (16₁ to 16ₙ) and the corresponding ultrasonic transducer elements (5₁ to 5ₙ).

19. Device according to Claim 18, whose driver stages (15₁ to 15ₙ) are connected to a power supply unit (30) whose output voltage can be set by means of the control unit (20).

20. Device according to one of Claims 1 to 19, which has monitoring means which measure the voltage and/or current at the outputs of the driver stages (15₁ to 15ₙ) and/or at the outputs of the signal conversion means and/or at the output of the power supply unit (30), and which compare said voltage and/or current with a corresponding reference value or corresponding reference values.

21. Device according to one of Claims 1 to 20, whose control unit (20) writes deviating bit patterns from the remainder of the memories into the memories (16₁ and 16₂, 16ₙ₋₁ and 16ₙ) belonging to ultrasonic transducer elements (5₁ and 5₂, 5ₙ₋₁ and 5ₙ) positioned in the edge region of the ultrasonic transducer (5), in such a way that the ultrasonic waves emitted by the ultrasonic transducer elements (5₁ and 5₂, 5ₙ₋₁ and 5ₙ) positioned in the edge region of the ultrasonic transducer (5) have a reduced intensity in comparison to the ultrasonic waves emitted by the remainder of the ultrasonic transducer elements.

22. Device according to Claim 21, whose control unit (20) writes bit patterns into the memories belonging to ultrasonic transducer elements not positioned in the edge region of the ultrasonic transducer (5), each of said bit patterns containing a sequence of immediately successive set bits, and said control unit writes bit patterns into the memories (16₁ and 16₂, 16ₙ₋₁ and 16ₙ) belonging to ultrasonic transducer elements (5₁ and 5₂, 5ₙ₋₁ and 5ₙ) positioned in the edge region of the ultrasonic transducer (5), said bit patterns containing a reduced number of successive set bits.

## Revendications

1. Dispositif pour traiter un objet à l'aide d'ondes ultrasonores focalisées, comportant un convertisseur d'ultrasons (5) composé de plusieurs éléments convertisseurs d'ultrasons (5₁ à 5ₙ), caractérisé par un dispositif qui est destiné à commander le convertisseur d'ultrasons (5) et qui comporte pour chaque élément convertisseur d'ultrasons (5₁ à 5ₙ) une mémoire (16₁ à 16ₙ) dans laquelle une configuration binaire correspondant à une position souhaitée du foyer (F) des ondes ultrasonores est mémorisée au cours du fonctionnement et par une unité de commande (20) qui adresse en parallèle et de façon cyclique les mémoires (16₁ à 16ₙ) de telle sorte qu'il apparaît aux sorties de données des mémoires (16₁ à 16ₙ) des signaux électriques qui sont décalés dans le temps les uns par rapport aux autres en fonction de la position souhaitée du foyer (F) et qui sont envoyés à l'élément convertisseur d'ultrasons (5₁ à 5ₙ) concerné.

2. Dispositif selon la revendication 1, qui contient comme mémoires (16₁ à 16ₙ) des mémoires à lecture et écriture dans lesquelles l'unité de commande (20) écrit les configurations binaires correspondant à la position souhaitée du foyer (F).

3. Dispositif selon la revendication 1 ou 2, dont l'unité de commande (20) calcule les configurations binaires correspondant à la position souhaitée du foyer (F) et les écrit dans les mémoires (16₁ à 16ₙ).

4. Dispositif selon l'une des revendications 1 à 3, dont l'unité de commande (20) comporte une mémoire de données (28) dans laquelle des configurations binaires pour des positions différentes du foyer (F) sont mémorisées, l'unité de commande (20) écrivant à chaque fois dans les mémoires (16₁ à 16ₙ) les configurations binaires correspondant à la position souhaitée du foyer (F).

5. Dispositif selon la revendication 4, dans la mémoire de données (28) duquel sont mémorisées des configurations binaires pour différentes positions du foyer (F) qui sont disposées en forme de matrice à ceux ou trois dimensions.

6. Dispositif selon l'une des revendications 1 à 5, dans lequel des moyens d'entrée (18, 35) pour spécifier une position souhaitée du foyer (F) sont associés à l'unité de commande (20).

7. Dispositif selon la revendication 6, qui comporte un dispositif de localisation, au moyen duquel une image d'au moins une zone de l'objet à traiter peut être produite, et dont les moyens d'entrée (18, 35) sont conçus de telle sorte que la position souhaitée du foyer (F) peut être choisie dans l'image.

8. Dispositif selon la revendication 7, qui comporte comme dispositif de localisation un dispositif de localisation à ultrasons (5, 13, 17).

9. Dispositif selon la revendication 8, dont le dispositif de localisation à ultrasons (5, 13, 17) produit une image en coupe d'au moins une zone de l'objet à traiter, les positions différentes du foyer (F) mémorisées dans la mémoire de données (28) se trouvant dans un plan qui est contenu dans la zone, projetée dans l'image en coupe, de l'objet à traiter.

10. Dispositif selon la revendication 8 ou 9, dont le convertisseur d'ultrasons (5) fait partie du dispositif de localisation à ultrasons (5, 13, 17).

11. Dispositif selon l'une des revendications 1 à 10, dont le convertisseur d'ultrasons (5) contient un agencement linéaire (linear array) d'éléments convertisseurs d'ultrasons (5₁ à 5ₙ).

12. Dispositif selon les revendications 10 et 11, dans lequel le convertisseur d'ultrasons (5) permet d'explorer l'objet à traiter pour produire une image en coupe à ultrasons lors d'une opération d'exploration par ligne (linear scan).

13. Dispositif selon l'une des revendications 1 à 12, qui comporte des moyens de transformation de signaux qui sont branchés entre les sorties de données des mémoires (16₁ à 16ₙ) et les éléments convertisseurs d'ultrasons (5₁ à 5ₙ) correspondants et qui transforment les signaux rectangulaires apparaissant aux sorties de données des mémoires (16₁ à 16ₙ) en des signaux au moins globalement sinusoïdaux.

14. Dispositif selon la revendication 13, dont les moyens de transformation de signaux sont des circuits LC (14₁ à 14ₙ).

15. Dispositif selon l'une des revendications 1 à 14, dont les mémoires (16₁ à 16ₙ) ont chacune une profondeur de mémoire de 1 bit.

16. Dispositif selon l'une des revendications 1 à 15, dont les mémoires (16₁ à 16ₙ) ont chacune une longueur de mémoire de 16 bits.

17. Dispositif selon l'une des revendications 1 à 16, dans lequel la fréquence d'horloge avec laquelle l'unité de commande (20) adresse les mémoires (16₁ à 16ₙ) est égale à la fréquence des ondes ultrasonores multipliée par la longueur de mémoire.

18. Dispositif selon l'une des revendications 1 à 17, qui comporte des étages d'attaque (15₁ à 15ₙ) branchés entre les sorties de données des mémoires (16₁ à 16ₙ) et les éléments convertisseurs d'ultrasons (5₁ à 5ₙ) correspondants.

19. Dispositif selon la revendication 18, dont les étages d'attaque (15₁ à 15ₙ) sont reliés à une unité d'alimentation en courant (30) dont la tension de sortie peut être réglée au moyen de l'unité de commande (20).

20. Dispositif selon l'une des revendications 1 à 19, qui comporte des moyens de contrôle qui mesurent la tension et/ou le courant aux sorties des étages d'attaque (15₁ à 15ₙ) et/ou aux sorties des moyens de transformation de signaux et/ou à la sortie de l'alimentation en courant (30) et qui comparent cette ou ces valeurs mesurées à une ou des valeurs de référence correspondantes.

21. Dispositif selon l'une des revendications 1 à 20, dont l'unité de commande (20) écrit dans les mémoires (16₁ et 16₂, 16ₙ₋₁ et 16ₙ) associées à des éléments convertisseurs d'ultrasons (5₁ et 5₂, 5ₙ₋₁ et 5ₙ) se trouvant dans la zone du bord du convertisseur d'ultrasons (5) des configurations binaires s'écartant de celles des autres mémoires de telle sorte que les ondes ultrasonores partant des éléments convertisseurs d'ultrasons (5₁ et 5₂, 5ₙ₋₁ et 5ₙ) se trouvant dans la zone du bord du convertisseur d'ultrasons (5) ont une intensité réduite en comparaison de celle des ondes ultrasonores partant des autres éléments convertisseurs d'ultrasons.

22. Dispositif selon la revendication 21, dont l'unité de commande (20) écrit dans les mémoires associées à des éléments convertisseurs d'ultrasons ne se trouvant pas dans la zone du bord du convertisseur d'ultrasons (5) des configurations binaires qui contiennent chacune une suite de bits immédiatemant consécutifs affectés, et dans les mémoires (16₁ et 16₂, 16ₙ₋₁ et 16ₙ) associées à des éléments convertisseurs d'ultrasons (5₁ et 5₂, 5ₙ₋₁ et 5ₙ) se trouvant dans la zone du bord du convertisseur d'ultrasons (5), des configurations binaires qui contiennent un nombre réduit de bits consécutifs affectés.
